# EUROPEAN PATENT SPECIFICATION

(11) **EP 4 392 652 B1**
(45) Date of publication and mention of the grant of the patent: **06.08.2025**
(21) Application number: 22768896.7
(22) Date of filing: 19.08.2022
(51) Int. Cl.: F02D 33/00, F02M 37/10, G01N 33/28, B60K 15/00, B60K 15/03

(54) **A FUEL MONITORING SYSTEM**
KRAFTSTOFFÜBERWACHUNGSSYSTEM
SYSTÈME DE SURVEILLANCE DE CARBURANT

(30) Priority: 23.08.2021 GB 202112085
(43) Date of publication of application: 03.07.2024
(73) Proprietor: Fuel Active Limited, Cardiff, South Glamorgan CF15 7JD (GB)
(72) Inventor: HUGHES, Tomas Edward, Taffs Well, Cardiff CF15 7JD (GB); JOHNS, Curtis, Taffs Well, Cardiff CF15 7JD (GB); MASSEY, Nicholas James Andrew, Taffs Well, Cardiff CF15 7JD (GB); MIDDLETON, Thomas, Taffs Well, Cardiff CF15 7JD (GB); THOMAS, Jack Ellis, Taffs Well, Cardiff CF15 7JD (GB)
(74) Representative: Definition IP Limited
(86) International application number: PCT/GB2022/052159
(87) International publication number: WO 2023/026028

(56) References cited:
- WO-A1-2020/257434
- US-A- 5 973 503
- US-A1- 2003 140 715
- US-A1- 2017 248 094

## Description

A fuel supply arrangement associated with a fuel monitoring system is for instance known from US 2017/248094 A1. The present invention relates to a fuel monitoring system for monitoring the properties of the fuel being consumed by, for example, an internal combustion engine, and for monitoring the environmental conditions in which that fuel is being consumed. The fuel monitoring system is therefore able to provide information on the quality of the fuel, in real time, as it is being consumed. It is advantageous to be able to monitor the properties of fuel and to determine the quality of the fuel, in real time, in order to mitigate the detrimental effects of endemic diesel fuel contamination and realise increases in operational efficiency, such as can be achieved through reduced fuel consumption and longer intervals between servicing of equipment.

Accordingly, the present invention provides a fuel supply arrangement, wherein the fuel supply arrangement comprises a fuel distribution arrangement and a fuel monitoring outlet line, wherein, in use, the fuel monitoring outlet line is connected to a source of fuel, wherein the fuel distribution arrangement comprises a fuel monitoring supply circuit connected at an upstream end to the fuel monitoring outlet line and comprising an inline fuel pump, wherein a first fuel sensor supply circuit and a second fuel sensor supply circuit are each connected to the fuel monitoring supply circuit downstream of the inline fuel pump, wherein the first fuel sensor supply circuit is provided with a sensor inlet connection and a sensor outlet connection for inline connection of a sensor, wherein the second fuel sensor supply circuit comprises a sensor manifold with ports for connection of a plurality of sensors in series with each other and wherein the fuel distribution arrangement comprises a fuel outlet line to which the first fuel sensor supply circuit and the second fuel sensor supply circuit are fluidly connected.

The provision of a first fuel sensor supply circuit and a second fuel sensor supply circuit is advantageous because it provides for flexibility in the characteristics of the fuel being provided to those circuits. The first and second fuel sensor supply circuits are arranged to accommodate fuel sensors of different types and those different types of fuel sensors have different requirements for the characteristics of the fuel that must be supplied to them, if those sensors are to operate properly and thus provide useful data. For example, the characteristics of the fuel that must be supplied to an inline particle contamination sensor are different to those that must be supplied to a water in oil sensor.

Preferably, the fuel monitoring supply circuit is connected between the fuel monitoring outlet line and a first port of a three port supply connector, the first fuel sensor supply circuit is connected to a second port of the three port supply connector and the second fuel supply circuit is connected to the third port of the three port supply connector, such that, the first fuel sensor supply circuit and the second fuel supply circuit are arranged in parallel. This parallel arrangement of the two separate fuel supply circuits is advantageous because it facilitates the provision to each supply circuit of a fuel supply with the characteristics that are required by the sensor or sensors located within those fuel circuits.

According to the invention, the fuel supply arrangement further comprises a fuel chamber having a fuel inlet for connection to the source of fuel and having a fuel monitoring outlet to which the fuel monitoring outlet line is connected.

The fuel chamber further comprises a fuel consumption outlet to which the fuel consumption outlet line is connected, for connection to a fuel consumption entity.

It is advantageous to have a fuel consumption outlet that is separate to the fuel monitoring outlet because in applications of the fuel supply arrangement of the present invention to fuel consumption entities that consume very large amounts of fuel, the flow rate of fuel through the fuel consumption outlet is so high that drawing a relatively small amount of fuel from that fuel consumption line along a fuel monitoring line for use by the fuel monitoring system would not be possible to achieve with the type of fuel pump that is otherwise needed to supply fuel within the fuel distribution arrangement.

Preferably, the fuel chamber is a sealed unit with the fuel inlet, the fuel consumption outlet and the fuel monitoring outlet line being the only openings in the fuel chamber.

Preferably, the fuel monitoring supply circuit comprises a fuel turbulence reduction loop which, in use, reduces the turbulence of fuel passing through the sensor inlet connection to a level required by a sensor connected to the sensor inlet connection. It is advantageous to provide fuel with a laminar flow for particular types of sensors, for example for an inline particle contamination sensor.

Preferably an inline particle contamination sensor is located between the sensor inlet connection and the sensor outlet connection of the first fuel sensor supply circuit.

Preferably, a moveable float is attached adjacent to the upstream pick-up end of a fuel pick-up tube and the downstream discharge end of the fuel pick-up tube is connected to the fuel inlet of the fuel chamber, wherein the vertical distance between the upstream pick-up end of the fuel pick-up tube and the downstream discharge end of the fuel pick-up tube can change with the level of fuel in the source of fuel. It is advantageous for the upstream pick-up end of the fuel pick-up tube to be located on a moveable float so that it is near to the surface of the fuel, rather than near to the base of the tank, because that reduces the chance of drawing fuel contaminated with debris into the pick-up tube.

Preferably, the fuel pick-up tube is flexible and the moveable float is retained within a float guide tube. It is advantageous to place the float within a guide tube to help to retain the float on the surface of the fuel, for example in instances when the present invention is fitted to a moving vehicle.

Preferably, the fuel chamber is located above a floating fuel pickup.

Preferably, the fuel chamber is located at least partially inside a fuel tank.

In an alternative arrangement, the fuel chamber is located externally to a fuel tank. In such a scenario the present invention may be provided with a large fuel pump, in order that fuel can still be drawn from the source of fuel, e.g. a fuel tank, to the fuel quality monitoring system.

Preferably, the fuel chamber is provided with a tubular inlet baffle located around a fuel inlet orifice, wherein the inlet baffle has a first end proximal to the fuel inlet orifice and wherein the first end is sealed to a wall of the fuel chamber, an open end distal to the fuel inlet orifice and spaced apart from a wall of the fuel chamber, a fuel consumption outlet with an inlet end that is adjacent to the open end of the inlet baffle and a fuel monitoring outlet with an inlet end that is adjacent to the first end of the inlet baffle. This arrangement is advantageous because by reducing the distance between the inlet end of the fuel monitoring outlet and the bottom of the fuel chamber the distance between any agitated air/fuel mixture at the top of the fuel chamber and the inlet end of the fuel monitoring outlet is increased, thus reducing the amount of air drawn into the fuel monitoring outlet. It is disadvantageous to draw air into the fuel monitoring outlet, because the presence of air in the fuel can reduce the accuracy of the sensors.

Preferably, a fuel/air space is located within the fuel chamber above the level of the inlet end of the fuel consumption outlet and below the top of the fuel chamber.

Preferably, the fuel monitoring system may comprise a fuel supply arrangement that further comprises at least one sensor connected to the fuel supply arrangement and a data acquisition sub-system connected to receive data from the at least one sensor.

Preferably, the data acquisition sub-system further comprises an analyser for at least partially analysing the data received from the at least one sensor and a data transmission means for transmitting the results of the analysis. This is advantageous because it can reduce the amount of data that needs to be transmitted wirelessly to an external receiver.

Preferably, the fuel monitoring system further comprises an inline particle contamination sensor provided in the first fuel sensor supply circuit for collecting data on the size and quantity of particulates in the fuel being monitored.

Preferably, the fuel monitoring system further comprises in the second fuel sensor supply circuit a fluid property sensor to detect the density, viscosity, temperature and dielectric constant of the fuel being monitored.

Preferably, the fuel monitoring system further comprises in the second fuel sensor supply circuit a sensor to detect the water content of the fuel being monitored.

Preferably, the fuel monitoring system further comprises a remotely located processing and display system connected to receive data from the data acquisition sub-system.

Preferably, the fuel monitoring system further comprises a cloud data storage system configured to receive data from the data acquisition sub-system and to transmit that data to the remotely located processing and display system.

The present invention will be described below, with reference to the following figures:
Figure 1 is a schematic view of a HGV tractor unit fitted with a fuel monitoring system according to the present invention;
Figure 2 is a schematic cross-sectional view of the fuel supply arrangement of the fuel monitoring system according to a first embodiment of the present invention with the HGV tractor unit of Figure 1 provided with a fuel chamber and a floating fuel pick-up;
Figure 3 is a detailed side perspective view of the fuel chamber, with the features located within it made visible by virtue of the fuel chamber wall being made transparent in the figure;
Figure 4 is a detailed top perspective view of the fuel chamber, with the features located within it made visible by virtue of the flange plate of the fuel chamber being made transparent in the figure;
Figure 5 is a schematic view of the fuel distribution arrangement; and
Figure 6 is a schematic cross-sectional view of the fuel supply arrangement of the fuel monitoring system according to an example not according to the appended claims with the fuel tank of the HGV tractor unit of Figure 1 provided with a simple fuel pick-up.

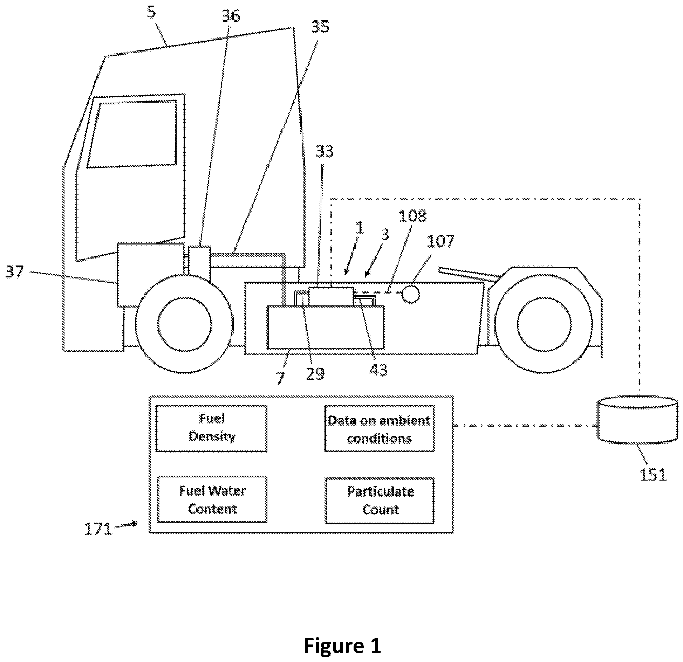

A fuel supply arrangement 1 for a fuel monitoring system 3 is shown in Figure 1. The fuel monitoring system 3 is fitted to a HGV tractor unit 5 and the fuel supply arrangement 1 is connected to its fuel tank 7, so that fuel can be drawn from the fuel tank 7 for monitoring. Data collected by the fuel monitoring system 3 is wirelessly transmitted to a remotely located processing and display system 171, via a cloud data storage system 151.

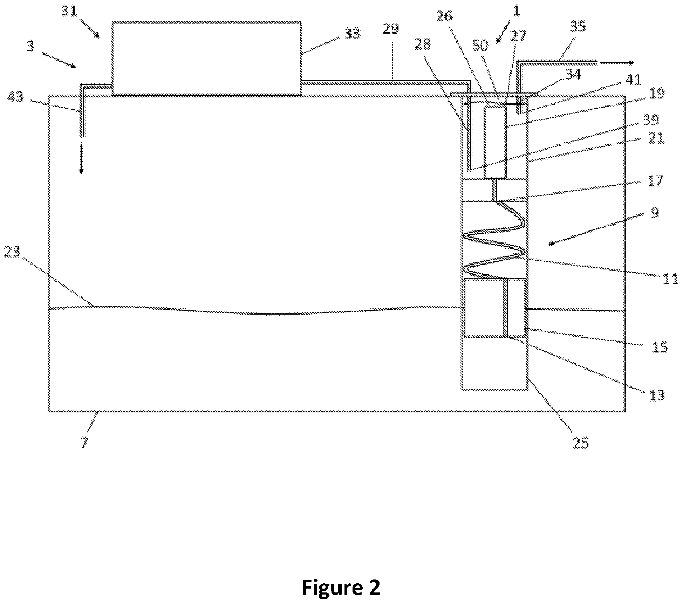

Figure 2 shows a first embodiment of the present invention with the fuel tank 7 in cross-section. The fuel supply arrangement 1 comprises a fuel pick-up arrangement 9 comprising an open-ended, flexible and helically wound, fuel pick-up line 11 with its upstream pick-up end 13 connected to a moveable float 15 and with its downstream discharge end 17 connected to an inlet baffle 19 of a fuel chamber 21. The moveable float 15 is located at the surface 23 of the fuel in the fuel tank 7 and moves up and down within a perforated float guide tube 25, as the amount of fuel in the fuel tank 7 changes. The inlet baffle 19 terminates at an open end 27 which is located in the upper half of the fuel chamber 21. A fuel monitoring outlet line 29 runs from a fuel monitoring outlet 28 in the interior of the fuel chamber 21 to a fuel distribution arrangement 31 located within an enclosure 33 that is positioned on top of the fuel tank 7. A fuel consumption outlet line 35 also runs from a fuel consumption outlet 34 in the interior of the fuel chamber 21 to an engine fuel pump 36 and then to the internal combustion engine 37 of the HGV tractor unit 5. The inlet end 39 of the fuel monitoring outlet 28 and the inlet end 41 of the fuel consumption outlet 34 are located lower than the outlet 27 of the inlet baffle 19. A fuel return line 43 is connected between the fuel distribution arrangement 31 and the fuel tank 7.

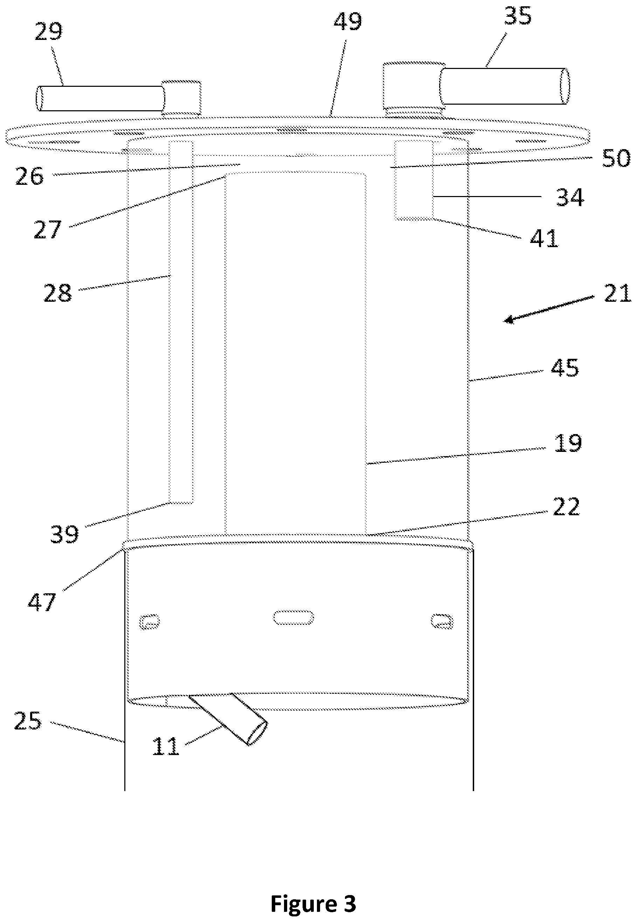

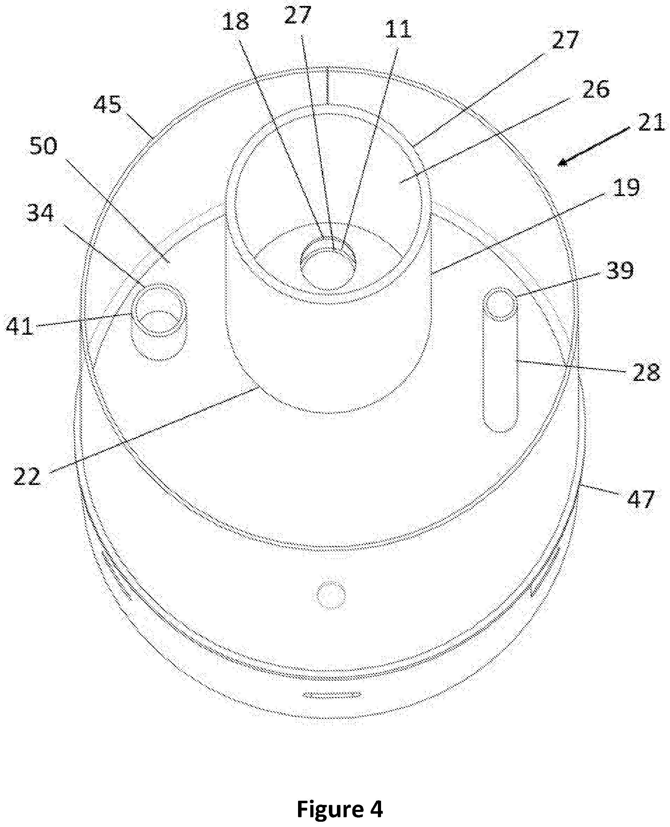

The fuel chamber 21 is shown in detail in Figure 3 and Figure 4. For the purposes of illustrating the features internal to the fuel chamber 21 the external wall 45 of the fuel chamber 21 has been made transparent in Figure 3 and the flange plate 49 has been made transparent in Figure 4. The external wall 45 of the fuel chamber 21 is in the form of a cylindrical hollow body with a circular base plate 47 closing its lower end and a circular flange plate 49 closing its upper end, with the base plate 47 and the flange plate 49 parallel to each other and perpendicular to the external wall 45. Thus, the fuel chamber 21 is a sealed unit except for the openings created for the inlet baffle 19, which extends vertically upwards into the fuel chamber 21 from the base plate 47, and for the fuel monitoring outlet 28 and the fuel consumption outlet 34 which each extend vertically downwards into the fuel chamber 21 from the flange plate 49.

The fuel monitoring outlet 28 extends from the interior of the fuel chamber 21 to the flange plate 49 and connects to the fuel monitoring outlet line 29, which is in turn connected to the fuel distribution arrangement 31. The fuel consumption outlet 34 also extends from the interior of the fuel chamber 21 to the flange plate 49 and it is connected to the fuel consumption outlet line 35, which is turn connected to the internal combustion engine 37. A fuel inlet orifice 18 (shown in Figure 4) is provided through the base plate 47 and the fuel pick-up tube 11 is connected to it. The inlet baffle 19 is an open-ended, solid walled, cylindrical tube (i.e. a tube which doesn't have any holes or perforations in its wall) which is located around the fuel inlet orifice 19 and is oriented perpendicularly relative to the base plate 47. The inlet baffle 19 is sealed to the base plate 47 at its lower end 22 and is open at its open end 27. The inlet baffle 19 has an internal diameter that is approximately four times the diameter of the fuel inlet orifice 18.

The inlet baffle 19 extends upwardly through the majority of the depth of the fuel chamber 21, but the open end 27 of the inlet baffle 19 is spaced from the underside of the flange plate 49, such that an inlet flow passage 26 is created between the inlet baffle 19 and the flange plate 49.

The fuel monitoring outlet 28 extends downwardly through the majority of the depth of the fuel chamber 21, but its inlet end 39 is spaced from the upper side of the base plate 47. The fuel consumption outlet 34 extends downwardly into the fuel chamber 21, but only by a short distance, such that its inlet end 41 is close to the underside of the flange plate 49. The vertical spacing between the inlet end 39 and the inlet end 41 is large relative to the internal vertical height of the fuel chamber 21 and, as can be seen from Figure 3, that vertical spacing can be more than two-thirds of the internal vertical height of the fuel chamber 21. The fuel monitoring outlet 28 and the fuel consumption outlet 34 are located on diametrically opposite sides of the fuel chamber 21 and close to the inside surface of the external wall 45, such that the horizontal spacing between the inlet end 39 and the inlet end 41 is large relative to the internal diameter of the fuel chamber 21 and, as can be seen from Figure 4, that horizontal spacing can be more than three-quarters of the internal diameter of the fuel chamber 21. A fuel/air space 50 is located within the internal volume of the fuel chamber 21 above the level of the inlet 41 of the fuel consumption outlet 34 and below the level of the underside of the flange plate 49. The volume of this fuel/air space 50 is thus determined in part by the length of the fuel consumption outlet 34 and the volume of the fuel/air space 50 can be tuned for any particular application of the present invention, for example by changing the length of the fuel consumption outlet 34.

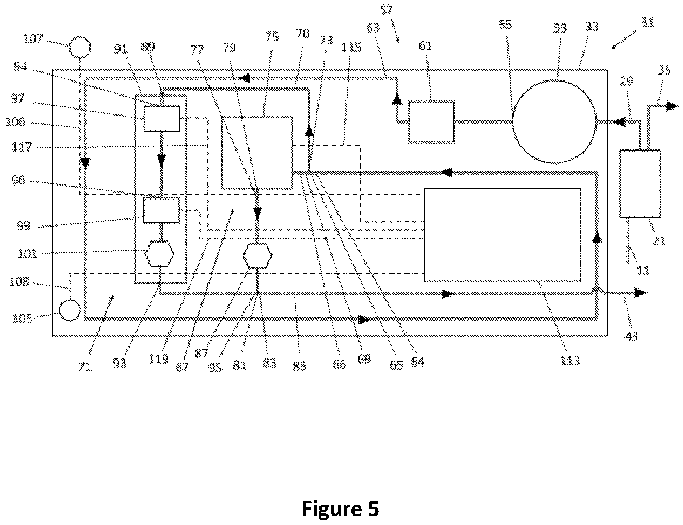

The fuel distribution arrangement 31 is shown schematically in Figure 5. The fuel monitoring outlet line 29 is attached to a fuel monitoring supply circuit 57 which comprises an inline fuel pump 53. A fuel strainer / filter 61 is located downstream of, and in close proximity to, the inline fuel pump 53. A fuel turbulence reduction loop 63 is located between the outlet of the fuel strainer / filter 61 and the inlet port 64 of a three port supply connector 65. The fuel turbulence reduction loop 63 runs around the internal perimeter of the enclosure 33 so that it has a length sufficient to reduce the turbulence of the fuel to a level which permits an inline particle contamination sensor 75 to accurately detect the number of particles per unit volume of fuel that is flowing through it. A fuel inlet line 66 of a particle detection circuit 67 is connected to the first outlet port 69 of the three port supply connector 65. A fuel inlet line 70 of a fuel property detection circuit 71 is connected to the second outlet port 73 of the three port connector 65.

The inline particle contamination sensor 75 is connected to the fuel inlet line 66 of the particle detection circuit 67, at a position adjacent to the first outlet port 69. A fuel return line 77 is provided in the particle detection circuit 67 between an outlet 79 of the inline particle contamination sensor 75 and an inlet port 81 of a three port return connector 83 of a fuel outlet circuit 85. The fuel outlet circuit 85 is connected to the fuel return line 43. A check valve 87 is located in the fuel return line 77, adjacent to the outlet 79.

An inlet 89 of a manifold block 91 is connected to the fuel inlet line 70 of the fuel property detection circuit 71. An outlet 93 of the manifold block 91 is connected to a second inlet port 95 of the three port return connector 83 of the fuel outlet circuit 85. The manifold block 91 provides a first connection 94 for inline series connection of a water in oil sensor 97, a second connection 96 for inline series connection of a fluid property sensor 99 and a third connection 100 for inline series connection of a check valve 101. The water in oil sensor 97, the fluid property sensor 99 and the check valve 101 are located between the inlet 89 and the outlet 93 of the manifold block 91, in series and in that order.

The enclosure 33 also contains a data acquisition, analysis and transmission data sub-system 113. Wired data connections 115, 117 and 119 connect the inline particle contamination sensor 75, the water in oil sensor 97 and the fluid property sensor 99 respectively to the data sub-system 113. Also located within the enclosure 33 and adjacent to its base is a proximity sensor 105, which is connected to the data sub-system 113 by a wired data connection 108. An ambient temperature and humidity sensor 107 is located outside of the enclosure 33 and connected to the data sub-system 113 by a wired data connection 106.

The data sub-system 113 communicates wirelessly with a cloud data storage system 151. A remotely located processing and display system 171 is also in wireless communication with the cloud data storage system 151. Data can be received by and sent from the cloud storage system 151 and both the data sub-system 113 and the remotely located processing and display system 171 can send and receive data. The remotely located processing and display system 171 is configured to display information collected by the various sensors and information resulting from analysis, such as can be undertaken by the data acquisition, analysis and transmission data sub-system 113 . For example, and as illustrated in Figure 1, the remotely located processing and display system 171 can display information about the fuel density (as detected by the fluid property sensor 99), the ambient conditions, such as temperature and humidity, (as detected by the ambient temperature and humidity sensor 107), the particulate count (as detected by the particle contamination sensor 75) and the fuel water content (as detected by the water in oil sensor 97).

In use of the fuel supply arrangement 1 on a HGV tractor unit 5, the engine fuel pump 36 provided on the internal combustion engine 37 draws fuel into the fuel chamber 21. The fuel is drawn through the upstream pick-up end 13 of the fuel pick-up tube 11 attached to the moveable float 15, flows through the fuel pick-up tube 11 and then out of its downstream discharge end 17, through the fuel inlet orifice 18 and then into the inlet baffle 19 of the fuel chamber 21. The fuel exits the open end 27 of the inlet baffle 19, flows into the fuel chamber 21 and flows out of the fuel chamber 21 to the internal combustion engine 37 through an inlet end 41 of the fuel consumption outlet 34. The fuel entering the fuel chamber 21 via the fuel inlet orifice 18 does not mix with the rest of the fuel in the fuel chamber 21 until it has passed through the height of the inlet baffle 19 and exited into the fuel chamber 21 through the inlet flow passage 26. The relatively large internal diameter of the baffle 19, compared to the diameter of the fuel inlet orifice 18, combined with the height of the inlet baffle 19, provides a significant volume through which the fuel must pass before it can enter into the fuel chamber 21, from where it can be extracted via the fuel monitoring outlet 28 or the fuel consumption outlet 34. In passing through the relatively large internal volume of the inlet baffle 19 the velocity of fuel entering the fuel chamber 21 is reduced, which leads to a reduction in the amount of agitation of the fuel and air mixture in the fuel / air space 50 at the top of the fuel chamber 21. A reduction in the agitation of the fuel and air mixture in the fuel / air space 50 has the advantage of reducing the amount of air being drawn into the fuel monitoring outlet line 29 and the into fuel consumption outlet line 35, which is beneficial because, for example, if air is present in the fuel passing through the fuel sensors of the fuel monitoring system 3, then the accuracy of the readings of those sensors can be negatively impacted.

Upon first use of the fuel supply arrangement 1, or use after a fuel system servicing operation, the fuel chamber 21 must be filled with fuel until the level of fuel in the fuel chamber 21 is above the level of the inlet end 39 of the fuel monitoring outlet 28 and above the level of the inlet end 41 of the fuel consumption outlet 34. This is achieved by a priming operation in which air is removed from the fuel chamber 21 (because the fuel chamber 21 is sealed) in order for fuel chamber 21 to fill with fuel. The engine 37 consumes fuel as it runs and the engine fuel pump 36 ensures that the level of fuel in the fuel chamber 21 remains at the correct level. Some air remains at the top of the fuel chamber, in the fuel / air space 50. The volume of air retained in the fuel chamber 21 is reduced by virtue of the relatively short length of the fuel consumption outlet 34.

To supply fuel to the fuel monitoring system 3, the inline fuel pump 53 of the fuel monitoring supply circuit 57 draws fuel from the fuel chamber 21 via the inlet end 39 of the fuel monitoring outlet 28. The inlet end 39 is drawing fuel from the fuel chamber 21 and thus the fuel being supplied to the fuel monitoring system 3 for analysis is representative of the fuel being consumed by the internal combustion engine 37 of the HGV tractor unit 5, because that fuel is also drawn from the fuel chamber 21.

The fuel flows through the pump 53, into the fuel monitoring supply circuit 57 through the fuel strainer / filter 61 and into the fuel turbulence reduction loop 63. The turbulence of the fuel is reduced as it passes through the fuel turbulence reduction loop 63 and before it reaches the inlet port 64 of the three port supply connector 65, where the fuel is split into a supply to the particle detection circuit 67 and a supply to the fuel property detection circuit 71. The pressure of fuel in the fuel monitoring supply circuit 57, in the fuel turbulence reduction loop 63 and consequently in the particle detection circuit 67, is controlled by the characteristics of the inline fuel pump 53 and the method in which the inline fuel pump 53 is driven, such that the pressure and volumetric flow of fuel can be maintained at the desired level (such as might be required for correct operation of the inline particle contamination sensor 75).

The fuel for the particle detection circuit 67 passes out of the first outlet port 69 of the three port connector 65 into the fuel inlet line 66 and then into the inline particle contamination sensor 75, where the number of particles per unit volume of fuel that is flowing through it is counted, and the size of those particles is measured. The fuel passing into the inline particle contamination sensor 75 has been filtered by the fuel strainer/filter 61 to prevent blockage of the inline particle contamination sensor 75. The data collected on the number of particles per unit volume and the size of the particles is transmitted by the wired data connection 115 to the data sub-system 113 for processing and onwards transmission to the remotely located processing and display system 171. Upon the fuel leaving the particle contamination sensor 75 through the outlet 79 that fuel flows to the check valve 87. The purpose of the check valve 87 is to maintain the pressure in the particle detection circuit 67 at a level that permits correct operation of the inline particle contamination sensor 75. The fuel flows through the check valve 87 to the three port return connector 83 and then to the fuel outlet circuit 85 from which the fuel then flows to the fuel return line 43 and back to the fuel tank 7.

The fuel for the fuel property detection circuit 71 passes out of the second outlet port 73 of the three port supply connector 65 and into the manifold block 91 through its inlet 89. The fuel flows through the water in oil sensor 97, then through the fluid property sensor 99 and then through the check valve 101, before passing out through the outlet 93 of the manifold block 91 to the three port return connector 83. The fuel then flows to the fuel outlet circuit 85 and then to the fuel return line 43 and back to the fuel tank 7. The data collected by the water in oil sensor 97 about the water content of the fuel is transmitted by the wired data connection 117 to the data sub-system 113 and the data collected by the fluid property sensor 99 about the density, viscosity, temperature and dielectric constant of the fuel is transmitted by the wired data connection 119 to the data sub-system 113 for processing and onwards transmission to the remotely located processing and display system 171.

The proximity sensor 105 detects the presence of fluid leaking into the enclosure 33, for example fuel from a loose connection within one of the fuel circuits of the fuel distribution arrangement 31, or water entering into the enclosure 33 from outside. If a fluid is detected by the proximity sensor 105 then it sends an electrical signal to the data sub-system 113 and that signal can be used to display a warning to, for example, an operator, via the remotely located processing and display system. The data sub-system 113 comprises a global positioning system (GPS), for example to locate the position of the HGV tractor unit 5 and to track its movements and the ambient temperature and humidity sensor 107 collects data about the environment in which the fuel supply arrangement 1 is located. The data from the proximity sensor 105 is transmitted to the data sub-system 113 by the wired data connection 108 and the data from the ambient temperature and humidity sensor 107 is transmitted to the data sub-system 113 by the wired data connection 106. The fuel monitoring system 3 may also be provided with a fuel level sensor (not shown), provided in the fuel tank 7. The fuel level sensor is capable of providing very accurate information about the amount of fuel in the tank 7. The additional information provided by this fuel sensor level can be particularly useful if the fuel monitoring system 3 is fitting to a fuel tank 7 which already has a pre-existing fuel level sensor, but that pre-existing fuel level sensor is not capable of providing information about the level of the fuel in the tank 7 with the desired level of accuracy.

The remotely located processing and display system 171 is monitored by an operator and that operator may be on the HGV tractor unit 5, or may be located remotely from the HGV tractor unit 5, for example if the HGV tractor unit 5 is an autonomous vehicle. A typical use of the data collected by the fuel monitoring system 3 is to alert the operator that an HGV tractor unit 5 is currently consuming sub-standard fuel. In certain circumstances the operator can be instructed to shut down the internal combustion engine 37 of the HGV tractor unit, for example to prevent it from being damaged.

The information collected by the various sensors and received by the remotely located processing and display system 171 can be utilised in various ways. For example, it can be sent to a monitoring centre, or it can be sent to a pre-existing dashboard, for example that of the HGV tractor unit 5, where the information can be displayed in the manner consistent with the general approach of the OEM manufacturer of the HGV tractor unit 5.

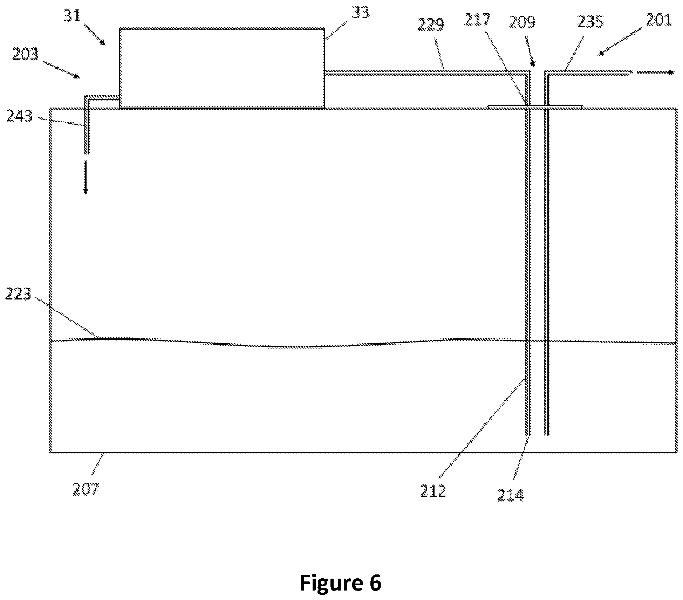

Figure 6 shows an example not according to the appended claims with a fuel tank 207 in cross-section. The fuel supply arrangement 201 comprises a fuel pick-up arrangement 209 comprising a straight, rigid, pick-up pipe 212 with its upstream pick-up end 214 located near to the bottom of the fuel tank 207 and with its downstream discharge end 217 connected to a fuel monitoring outlet line 229. In normal use, the pick-up end 214 remains below the surface 223 of the fuel in the tank 207.The fuel monitoring outlet line 229 runs to a fuel distribution arrangement 31 of a fuel monitoring system 203 which is located within an enclosure 33 that is positioned on top of the fuel tank 207. The fuel distribution arrangement 31 is as described above and as illustrated schematically in Figure 5.

A straight and rigid fuel consumption outlet line 235 also runs from a position near to the bottom of the fuel tank 207 vertically upwards, out of the tank 207 and to an internal combustion engine 37 of a HGV tractor unit 5, such as is shown in Figure 1. A fuel return line 243 is connected between the fuel distribution arrangement 31 and the fuel tank 207.

Operation of the fuel supply arrangement 201 according to the example is the same as that of the fuel supply arrangement 1 of the first embodiment, as described above, other than that fuel is drawn directly from the tank 207 by the inline fuel pump 53 of the fuel distribution arrangement 31, rather than being drawn from a separate chamber, e.g. the fuel chamber 21 of the first embodiment. In this example, the fuel being monitored by the fuel monitoring system 203 is representative of the fuel in the fuel tank 207, whereas in the first embodiment of the present invention the fuel being monitored by the fuel monitoring system 203 is representative of the fuel in the fuel chamber 21, i.e. fuel which has been collected from near the surface 23 of the fuel in the fuel tank 7, due to the upstream pick-up end 13 of the fuel pick-up tube 11 being attached to the moveable float 15.

## Claims

1. A fuel supply arrangement (1) for a fuel monitoring system (3) wherein the fuel supply arrangement (1) comprises a fuel distribution arrangement (31) and a fuel monitoring outlet line (29), wherein, in use, the fuel monitoring outlet line (29) is connected to a source of fuel (7), wherein the fuel distribution arrangement (31) comprises a fuel monitoring supply circuit (57) connected at an upstream end to the fuel monitoring outlet line (29) and comprising an inline fuel pump (53), wherein a first fuel sensor supply circuit (67) and a second fuel sensor supply circuit (71) are each connected to the fuel monitoring supply circuit (57) downstream of the inline fuel pump (53), wherein the first fuel sensor supply circuit (67) is provided with a sensor inlet connection and a sensor outlet connection for inline connection of a sensor, wherein the second fuel sensor supply circuit (71) comprises a sensor manifold (91) with ports for connection of a plurality of sensors (97,99) in series with each other and wherein the fuel distribution arrangement (31) comprises a fuel outlet line (43) to which the first fuel sensor supply circuit (67) and the second fuel sensor supply circuit (71) are fluidly connected, **characterised in that** the fuel supply arrangement (1) further comprises a fuel chamber (21) having a fuel inlet (19) for connection to the source of fuel (7) and having a fuel monitoring outlet (28) to which the fuel monitoring outlet line (29) is connected, wherein the fuel chamber (21) further comprises a fuel consumption outlet (34) to which a fuel consumption outlet line (35) is connected, for connection to a fuel consumption entity (37).

2. A fuel supply arrangement (1) as claimed in claim 1, wherein the fuel monitoring supply circuit (57) is connected between the fuel monitoring outlet line (29) and a first port of a three port supply connector (65), the first fuel sensor supply circuit (67) is connected to a second port of the three port supply connector (65) and the second fuel supply circuit (71) is connected to the third port of the three port supply connector (65), such that, the first fuel sensor supply circuit (67) and the second fuel supply circuit (71) are arranged in parallel.

3. A fuel supply arrangement (1) as claimed in claim 1 or claim 2, wherein the fuel chamber (21) is a sealed unit with the fuel inlet (19), the fuel consumption outlet (34) and the fuel monitoring outlet (28) being the only openings in the fuel chamber (21).

4. A fuel supply arrangement (1) as claimed in any preceding claim, wherein the fuel monitoring supply circuit (57) comprises a fuel turbulence reduction loop (63) which, in use, reduces the turbulence of fuel passing through the sensor inlet connection to a level required by a sensor connected to the sensor inlet connection.

5. A fuel supply arrangement (1) as claimed in any preceding claim, wherein an inline particle contamination sensor (75) is located between the sensor inlet connection and the sensor outlet connection of the first fuel sensor supply circuit (67).

6. A fuel supply arrangement (1) as claimed in any preceding claim, wherein a moveable float (15) is attached adjacent to the upstream pick-up end (13) of a fuel pick-up tube (11) and the downstream discharge end (17) of the fuel pick-up tube (11) is connected to the fuel inlet (19) of the fuel chamber (21) wherein the vertical distance between the upstream pick-up end (13) of the fuel pick-up tube (11) and the downstream discharge end (17) of the fuel pick-up tube (11) can change with the level of fuel in the source of fuel (7)

7. A fuel supply arrangement (1) as claimed in claim 6, wherein the fuel pick-up tube (11) is flexible and wherein the moveable float (15) is retained within a float guide tube (25).

8. A fuel supply arrangement (1) as claimed in any preceding claim, wherein the fuel chamber (21) is located above a floating fuel pickup.

9. A fuel supply arrangement (1) as claimed in any preceding claim in which the fuel chamber (21) is located at least partially inside a fuel tank (7).

10. A fuel supply arrangement (1) as claimed in any one of claims 1 to 8 in which the fuel chamber (21) is located externally to a fuel tank (7).

11. A fuel supply arrangement (1) as claimed in any preceding claim in which the fuel chamber (21) is provided with a tubular inlet baffle (19) located around a fuel inlet orifice (18), wherein the inlet baffle (19) has a first end (22) proximal to the fuel inlet orifice (18) and wherein the first end (22) is sealed to a wall of the fuel chamber (21), an open end (27) distal to the fuel inlet orifice (18) and spaced apart from a wall of the fuel chamber(21), a fuel consumption outlet (34) with an inlet end (41) that is adjacent to the open end (27) of the inlet baffle (19) and a fuel monitoring outlet (28) with an inlet end (39) that is adjacent to the first end (22) of the inlet baffle (19).

12. A fuel supply arrangement (1) as claimed in any preceding claim in which a fuel/air space (50) is located within the fuel chamber (21) above the level of the inlet end (41) of the fuel consumption outlet (34) and below the top of the fuel chamber (21).

13. A fuel monitoring system (3) comprising a fuel supply arrangement (1) as claimed in any one of claims 1 to 12 and further comprising at least one sensor (75,97,99) connected to the fuel supply arrangement (1) and a data acquisition sub-system (113) connected to receive data from the at least one sensor (75,97,99).

14. A fuel monitoring system (3) as claimed in claim 13 wherein the data acquisition sub-system (113) further comprises an analyser for at least partially analysing the data received from the at least one sensor (75,97,99) and a data transmission means for transmitting the results of the analysis.

15. A fuel monitoring system (3) as claimed in claim 13 or claim 14, further comprising an inline particle contamination sensor (75) provided in the first fuel sensor supply circuit (67) for collecting data on the size and quantity of particulates in the fuel being monitored.

16. A fuel monitoring system (3) as claimed in any one of claims 13, 14 or 15, further comprising in the second fuel sensor supply circuit (71) a fluid property sensor (99) to detect the density, viscosity, temperature and dielectric constant of the fuel being monitored.

17. A fuel monitoring system (3) as claimed in any one of claims 13 to 16, further comprising in the second fuel sensor supply circuit (71) a sensor (97) to detect the water content of the fuel being monitored.

18. A fuel monitoring system (3) as claimed in any one of claims 13 to 17, further comprising a remotely located processing and display system (171) connected to receive data from the data acquisition sub-system (113).

19. A fuel monitoring system (3) as claimed in claim 18, further comprising a cloud data storage system (151) configured to receive data from the data acquisition sub-system (113) and to transmit that data to the remotely located processing and display system (171).

## Patentansprüche

1. Kraftstoffversorgungsanordnung (1) für ein Kraftstoffüberwachungssystem (3), wobei die Kraftstoffversorgungsanordnung (1) eine Kraftstoffverteilungsanordnung (31) und eine Kraftstoffüberwachungs-Auslassleitung (29) umfasst, wobei die Kraftstoffüberwachungs-Auslassleitung (29) im Gebrauch mit einer Kraftstoffquelle (7) verbunden ist, wobei die Kraftstoffverteilungsanordnung (31) einen Kraftstoffüberwachungs-Versorgungskreis (57) umfasst, der an einem stromaufwärts gelegenen Ende mit der Kraftstoffüberwachungs-Auslassleitung (29) verbunden ist und eine Inline-Kraftstoffpumpe (53) umfasst, wobei ein erster Kraftstoffsensor-Versorgungskreis (67) und ein zweiter Kraftstoffsensor-Versorgungskreis (71) jeweils stromabwärts der Inline-Kraftstoffpumpe (53) mit dem Kraftstoffüberwachungs-Versorgungskreis (57) verbunden sind, wobei der erste Kraftstoffsensor-Versorgungskreis (67) mit einer Sensoreinlassverbindung und einer Sensorauslassverbindung zur Inline-Verbindung eines Sensors bereitgestellt ist, wobei der zweite Kraftstoffsensor-Versorgungskreis (71) einen Sensorverteiler (91) mit Anschlüssen zum Verbinden einer Vielzahl von Sensoren (97,99) in Reihe miteinander umfasst, und wobei die Kraftstoffverteilungsanordnung (31) eine Kraftstoffauslassleitung (43) umfasst, mit welcher der erste Kraftstoffsensor-Versorgungskreis (67) und der zweite Kraftstoffsensor-Versorgungskreis (71) strömungstechnisch verbunden sind, **dadurch gekennzeichnet, dass** die Kraftstoffversorgungsanordnung (1) weiter eine Kraftstoffkammer (21) umfasst, die einen Kraftstoffeinlass (19) zum Verbinden mit der Kraftstoffquelle (7) aufweist, und einen Kraftstoffüberwachungsauslass (28) aufweist, mit dem die Kraftstoffüberwachungs-Auslassleitung (29) verbunden ist, wobei die Kraftstoffkammer (21) weiter einen Kraftstoffverbrauchsauslass (34) umfasst, mit dem eine Kraftstoffverbrauchs-Auslassleitung (35) zur Verbindung mit einer Kraftstoffverbrauchseinheit (37) verbunden ist.

2. Kraftstoffversorgungsanordnung (1) nach Anspruch 1, wobei der Kraftstoffüberwachungs-Versorgungskreis (57) zwischen der Kraftstoffüberwachungs-Auslassleitung (29) und einem ersten Anschluss eines Versorgungsverbinders mit drei Anschlüssen (65) verbunden ist, der erste Kraftstoffsensor-Versorgungskreis (67) mit einem zweiten Anschluss des Versorgungsverbinders mit drei Anschlüssen (65) verbunden ist und der zweite Kraftstoffversorgungskreis (71) mit dem dritten Anschluss des Versorgungsverbinders mit drei Anschlüssen (65) verbunden ist, sodass der erste Kraftstoffsensor-Versorgungskreis (67) und der zweite Kraftstoffversorgungskreis (71) parallel angeordnet sind.

3. Kraftstoffversorgungsanordnung (1) nach Anspruch 1 oder Anspruch 2, wobei die Kraftstoffkammer (21) eine versiegelte Einheit mit dem Kraftstoffeinlass (19) ist, wobei der Kraftstoffverbrauchsauslass (34) und der Kraftstoffüberwachungsauslass (28) die einzigen Öffnungen in der Kraftstoffkammer (21) sind.

4. Kraftstoffversorgungsanordnung (1) nach einem vorstehenden Anspruch, wobei der Kraftstoffüberwachungs-Versorgungskreis (57) eine Kraftstoffturbulenz-Reduktionsschleife (63) umfasst, die im Gebrauch die Turbulenz von Kraftstoff, der durch die Sensoreinlassverbindung tritt, auf ein Niveau reduziert, das für einen Sensor, der mit der Sensoreinlassverbindung verbunden ist, erforderlich ist.

5. Kraftstoffversorgungsanordnung (1) nach einem vorstehenden Anspruch, wobei sich zwischen der Sensoreinlassverbindung und der Sensorauslassverbindung des ersten Kraftstoffsensor-Versorgungskreises (67) ein Inline-Partikelverunreinigungssensor (75) befindet.

6. Kraftstoffversorgungsanordnung (1) nach einem vorstehenden Anspruch, wobei ein beweglicher Schwimmer (15) an das stromaufwärts gelegene Aufnahmeende (13) eines Kraftstoffaufnahmerohrs (11) angrenzend angebracht ist und das stromabwärts gelegene Austragsende (17) des Kraftstoffaufnahmerohrs (11) mit dem Kraftstoffeinlass (19) der Kraftstoffkammer (21) verbunden ist, wobei sich der vertikale Abstand zwischen dem stromaufwärts gelegenen Aufnahmeende (13) des Kraftstoffaufnahmerohrs (11) und dem stromabwärts gelegenen Austragsende (17) des Kraftstoffaufnahmerohrs (11) mit dem Kraftstoffpegel in der Kraftstoffquelle (7) ändern kann.

7. Kraftstoffversorgungsanordnung (1) nach Anspruch 6, wobei das Kraftstoffaufnahmerohr (11) flexibel ist und wobei der bewegliche Schwimmer (15) innerhalb eines Schwimmerführungsrohres (25) gehalten wird.

8. Kraftstoffversorgungsanordnung (1) nach einem vorstehenden Anspruch, wobei sich die Kraftstoffkammer (21) oberhalb einer schwimmenden Kraftstoffaufnahme befindet.

9. Kraftstoffversorgungsanordnung (1) nach einem vorstehenden Anspruch, wobei sich die Kraftstoffkammer (21) mindestens teilweise innerhalb eines Kraftstofftanks (7) befindet.

10. Kraftstoffversorgungsanordnung (1) nach einem der Ansprüche 1 bis 8, wobei sich die Kraftstoffkammer (21) außerhalb eines Kraftstofftanks (7) befindet.

11. Kraftstoffversorgungsanordnung (1) nach einem vorstehenden Anspruch, wobei die Kraftstoffkammer (21) mit einer rohrförmigen Einlassdrossel (19), die sich um eine Kraftstoffeinlassöffnung (18) herum befindet, wobei die Einlassdrossel (19) ein erstes Ende (22) proximal zur Kraftstoffeinlassöffnung (18) aufweist und wobei das erste Ende (22) an einer Wand der Kraftstoffkammer (21) versiegelt ist, einem offenen Ende (27) distal zur Kraftstoffeinlassöffnung (18) und von einer Wand der Kraftstoffkammer (21) beabstandet, einem Kraftstoffverbrauchsauslass (34) mit einem Einlassende (41), das an das offene Ende (27) der Einlassdrossel (19) angrenzt, und einem Kraftstoffüberwachungsauslass (28) mit einem Einlassende (39), das an das erste Ende (22) der Einlassdrossel (19) angrenzt, bereitgestellt ist.

12. Kraftstoffversorgungsanordnung (1) nach einem vorstehenden Anspruch, wobei sich ein Kraftstoff-/Luft-Raum (50) innerhalb der Kraftstoffkammer (21) oberhalb des Niveaus des Einlassendes (41) des Kraftstoffverbrauchsauslasses (34) und unterhalb der Oberseite der Kraftstoffkammer (21) befindet.

13. Kraftstoffüberwachungssystem (3), das eine Kraftstoffversorgungsanordnung (1) nach einem der Ansprüche 1 bis 12 umfasst und weiter mindestens einen Sensor (75,97,99), der mit der Kraftstoffversorgungsanordnung (1) verbunden ist, und ein Datenerfassungs-Teilsystem (113) umfasst, das verbunden ist, um Daten von dem mindestens einen Sensor (75,97,99) zu empfangen.

14. Kraftstoffüberwachungssystem (3) nach Anspruch 13, wobei das Datenerfassungs-Teilsystem (113) weiter einen Analysator zum mindestens teilweisen Analysieren der von dem mindestens einen Sensor (75,97,99) empfangenen Daten und Datenübertragungsmittel zum Übertragen der Ergebnisse der Analyse umfasst.

15. Kraftstoffüberwachungssystem (3) nach Anspruch 13 oder Anspruch 14, das weiter einen Inline-Partikelverunreinigungssensor (75), der im ersten Kraftstoffsensor-Versorgungskreis (67) bereitgestellt ist, zum Sammeln von Daten über die Größe und Menge von Partikeln im überwachten Kraftstoff umfasst.

16. Kraftstoffüberwachungssystem (3) nach einem der Ansprüche 13, 14 oder 15, das weiter im zweiten Kraftstoffsensor-Versorgungskreis (71) einen Fluideigenschaftensensor (99) umfasst, um die Dichte, Viskosität, Temperatur und Dielektrizitätskonstante des überwachten Kraftstoffs zu detektieren.

17. Kraftstoffüberwachungssystem (3) nach einem der Ansprüche 13 bis 16, das weiter im zweiten Kraftstoffsensor-Versorgungskreis (71) einen Sensor (97) umfasst, um den Wassergehalt des überwachten Kraftstoffs zu detektieren.

18. Kraftstoffüberwachungssystem (3) nach einem der Ansprüche 13 bis 17, das weiter ein sich entfernt befindliches Verarbeitungs- und Anzeigesystem (171) umfasst, das verbunden ist, um Daten vom Datenerfassungs-Teilsystem (113) zu empfangen.

19. Kraftstoffüberwachungssystem (3) nach Anspruch 18, das weiter ein Cloud-Datenspeichersystem (151) umfasst, das konfiguriert ist, um Daten vom Datenerfassungs-Teilsystem (113) zu empfangen und diese Daten zum entfernt befindlichen Verarbeitungs- und Anzeigesystem (171) zu übertragen.

## Revendications

1. Ensemble d'alimentation en carburant (1) pour un système de surveillance de carburant (3), dans lequel l'ensemble d'alimentation en carburant (1) comprend un ensemble de distribution de carburant (31) et une conduite de sortie de surveillance de carburant (29), dans lequel, lors de l'utilisation, la conduite de sortie de surveillance de carburant (29) est raccordée à une source de carburant (7), dans lequel, l'ensemble de distribution de carburant (31) comprend un circuit d'alimentation en et de surveillance de carburant (57) raccordé au niveau d'une extrémité amont à la conduite de sortie de surveillance de carburant (29) et comprenant une pompe à carburant en ligne (53), dans lequel un premier circuit d'alimentation à capteur de carburant (67) et un second circuit d'alimentation à capteur de carburant (71) sont chacun raccordés au circuit d'alimentation en et de surveillance de carburant (57) en aval de la pompe à carburant en ligne (53), dans lequel le premier circuit d'alimentation à capteur de carburant (67) est doté d'un raccordement d'entrée de capteur et d'un raccordement de sortie de capteur pour le raccordement en ligne d'un capteur, dans lequel le second circuit d'alimentation à capteur de carburant (71) comprend un collecteur de capteur (91) avec des orifices pour le raccordement d'une pluralité de capteurs (97, 99) en série les uns avec les autres, et dans lequel l'ensemble de distribution de carburant (31) comprend une conduite de sortie de carburant (43) à laquelle le premier circuit d'alimentation à capteur de carburant (67) et le second circuit d'alimentation à capteur de carburant (71) sont raccordés fluidiquement, **caractérisé en ce que** l'ensemble d'alimentation en carburant (1) comprend en outre une chambre de carburant (21) présentant une entrée de carburant (19) pour le raccordement à la source de carburant (7) et présentant une sortie de surveillance de carburant (28) à laquelle la conduite de sortie de surveillance de carburant (29) est raccordée, dans lequel la chambre de carburant (21) comprend en outre une sortie de consommation de carburant (34) à laquelle une conduite de sortie de consommation de carburant (35) est raccordée, pour le raccordement à une entité de consommation de carburant (37).

2. Ensemble d'alimentation en carburant (1) selon la revendication 1, dans lequel le circuit d'alimentation en et de surveillance de carburant (57) est raccordé entre la conduite de sortie de surveillance de carburant (29) et un premier orifice d'un raccord d'alimentation à trois orifices (65), le premier circuit d'alimentation à capteur de carburant (67) est raccordé à un deuxième orifice du raccord d'alimentation à trois orifices (65) et le second circuit d'alimentation en carburant (71) est raccordé au troisième orifice du raccord d'alimentation à trois orifices (65), de telle sorte que le premier circuit d'alimentation à capteur de carburant (67) et le second circuit d'alimentation en carburant (71) soient agencés en parallèle.

3. Ensemble d'alimentation en carburant (1) selon la revendication 1 ou la revendication 2, dans lequel la chambre de carburant (21) est une unité scellée avec l'entrée de carburant (19), la sortie de consommation de carburant (34) et la sortie de surveillance de carburant (28) étant les seules ouvertures dans la chambre de carburant (21).

4. Ensemble d'alimentation en carburant (1) selon une quelconque revendication précédente, dans lequel le circuit d'alimentation en et de surveillance de carburant (57) comprend une boucle de réduction de turbulence de carburant (63) qui, lors de l'utilisation, réduit la turbulence du carburant passant à travers le raccordement d'entrée de capteur à un niveau requis par un capteur raccordé au raccord d'entrée de capteur.

5. Ensemble d'alimentation en carburant (1) selon une quelconque revendication précédente, dans lequel un capteur de contamination particulaire en ligne (75) est situé entre le raccord d'entrée de capteur et le raccord de sortie de capteur du premier circuit d'alimentation à capteur de carburant (67).

6. Ensemble d'alimentation en carburant (1) selon une quelconque revendication précédente, dans lequel un flotteur mobile (15) est fixé de manière adjacente à l'extrémité d'aspiration amont (13) d'un tube d'aspiration de carburant (11) et l'extrémité de refoulement aval (17) du tube d'aspiration de carburant (11) est raccordée à l'entrée de carburant (19) de la chambre de carburant (21), dans lequel la distance verticale entre l'extrémité d'aspiration amont (13) et l'extrémité de refoulement aval (17) du tube d'aspiration de carburant (11) peut varier en fonction du niveau de carburant dans la source de carburant (7).

7. Ensemble d'alimentation en carburant (1) selon la revendication 6, dans lequel le tube d'aspiration de carburant (11) est flexible et dans lequel le flotteur mobile (15) est retenu dans un tube de guidage de flotteur (25).

8. Ensemble d'alimentation en carburant (1) selon une quelconque revendication précédente, dans lequel la chambre de carburant (21) est située au-dessus d'une aspiration de carburant flottante.

9. Ensemble d'alimentation en carburant (1) selon une quelconque revendication précédente, dans lequel la chambre de carburant (21) est située au moins partiellement à l'intérieur d'un réservoir de carburant (7).

10. Ensemble d'alimentation en carburant (1) selon l'une quelconque des revendications 1 à 8, dans lequel la chambre de carburant (21) est située à l'extérieur d'un réservoir de carburant (7).

11. Ensemble d'alimentation en carburant (1) selon une quelconque revendication précédente, dans lequel la chambre de carburant (21) est dotée d'un déflecteur d'entrée tubulaire (19) situé autour d'un trou d'entrée de carburant (18), dans lequel le déflecteur d'entrée (19) présente une première extrémité (22) à proximité du trou d'entrée de carburant (18) et dans lequel la première extrémité (22) est scellée à une paroi de la chambre de carburant (21), une extrémité ouverte (27) distale par rapport au trou d'entrée de carburant (18) et espacée d'une paroi de la chambre de carburant (21), une sortie de consommation de carburant (34) avec une extrémité d'entrée (41) qui est adjacente à l'extrémité ouverte (27) du déflecteur d'entrée (19) et une sortie de surveillance de carburant (28) avec une extrémité d'entrée (39) qui est adjacente à la première extrémité (22) du déflecteur d'entrée (19).

12. Ensemble d'alimentation en carburant (1) selon une quelconque revendication précédente, dans lequel un espace carburant/air (50) est situé à l'intérieur de la chambre de carburant (21) au-dessus du niveau de l'extrémité d'entrée (41) de la sortie de consommation de carburant (34) et au-dessous de la partie supérieure de la chambre de carburant (21).

13. Système de surveillance de carburant (3) comprenant un ensemble d'alimentation en carburant (1) selon l'une quelconque des revendications 1 à 12 et comprenant en outre au moins un capteur (75, 97, 99) raccordé à l'ensemble d'alimentation en carburant (1) et un sous-système d'acquisition de données (113) connecté pour recevoir des données provenant du au moins un capteur (75, 97, 99).

14. Système de surveillance de carburant (3) selon la revendication 13, dans lequel le sous-système d'acquisition de données (113) comprend en outre un analyseur pour analyser au moins partiellement les données reçues du au moins un capteur (75, 97, 99) et un moyen de transmission de données pour transmettre les résultats de l'analyse.

15. Système de surveillance de carburant (3) selon la revendication 13 ou la revendication 14, comprenant en outre un capteur de contamination particulaire en ligne (75) disposé dans le premier circuit d'alimentation à capteur de carburant (67) pour collecter des données sur la taille et la quantité de particules dans le carburant surveillé.

16. Système de surveillance de carburant (3) selon l'une quelconque des revendications 13, 14 ou 15, comprenant en outre dans le second circuit d'alimentation à capteur de carburant (71) un capteur de propriété de fluide (99) pour détecter la densité, la viscosité, la température et la constante diélectrique du carburant surveillé.

17. Système de surveillance de carburant (3) selon l'une quelconque des revendications 13 à 16, comprenant en outre dans le second circuit d'alimentation à capteur de carburant (71) un capteur (97) pour détecter la teneur en eau du carburant surveillé.

18. Système de surveillance de carburant (3) selon l'une quelconque des revendications 13 à 17, comprenant en outre un système de traitement et d'affichage situé à distance (171) connecté pour recevoir des données du sous-système d'acquisition de données (113).

19. Système de surveillance de carburant (3) selon la revendication 18, comprenant en outre un système de stockage de données en nuage (151) configuré pour recevoir des données du sous-système d'acquisition de données (113) et pour transmettre ces données au système de traitement et d'affichage situé à distance (171).
